# EUROPEAN PATENT SPECIFICATION

(11) **EP 1 476 757 B1**
(45) Date of publication and mention of the grant of the patent: **07.01.2009**
(21) Application number: 02700861.4
(22) Date of filing: 19.02.2002
(51) Int. Cl.: G01N 33/574, G01N 33/52, G01N 33/558, G01N 33/543

(54) **METHOD FOR PREDICTING STOMACH CANCER**
VERFAHREN ZUR MAGENKREBSVORHERSAGE
PROCEDE POUR LA PREDICTION DU CANCER DE L'ESTOMAC

(43) Date of publication of application: 17.11.2004
(73) Proprietor: Zarita Biotech Co., Ltd., Suwon-Si, Kyonggi-Do 441-853 (KR); Woo, Hee-Jong, Seoul 137-070 (KR)
(72) Inventor: WOO, Hee-Jong, Seoul 137-070 (KR)
(74) Representative: Jappy, John William Graham
(86) International application number: PCT/KR2002/000249
(87) International publication number: WO 2003/071279

(56) References cited:
- US-A- 5 801 002
- US-A- 5 837 493
- US-A- 5 869 289
- US-A- 6 146 849
- US-B1- 6 281 333
- IURISCI I ET AL: "Concentrations of galectin-3 in the sera of normal controls and cancer patients." CLINICAL CANCER RESEARCH : AN OFFICIAL JOURNAL OF THE AMERICAN ASSOCIATION FOR CANCER RESEARCH. APR 2000, vol. 6, no. 4, April 2000 (2000-04), pages 1389-1393, XP002369298 ISSN: 1078-0432
- WOO H -J ET AL: "Immunohistochemical detection of galectin-3 in canine gastric carcinomas" JOURNAL OF COMPARATIVE PATHOLOGY, vol. 124, no. 2-3, February 2001 (2001-02), pages 216-218, XP002369299 ISSN: 0021-9975
- LEE JAY-YOUNG ET AL: "Galectin-3 expression in the development of gastric carcinomas" MOLECULAR BIOLOGY OF THE CELL, vol. 10, no. SUPPL., November 1999 (1999-11), page 272a, XP008060461 & 39TH ANNUAL MEETING OF THE AMERICAN SOCIETY FOR CELL BIOLOGY; WASHINGTON, D.C., USA; DECEMBER 11-15, 1999 ISSN: 1059-1524
- JARATLI H ET AL: "Galectin-3 and CD44v6 expression in early (T1a and T1b) breast cancer" LABORATORY INVESTIGATION, vol. 82, no. 1, January 2002 (2002-01), page 39A, XP008060460 & 2002 ANNUAL MEETING OF THE UNITED STATES AND CANADIAN ACADEMY OF PATHOLOGY; CHICAGO, IL, USA; FEBRUARY 23-MARCH 01, 2002 ISSN: 0023-6837
- KIM BYOUNG-SUCK ET AL: "Correlations between galectin-3 expression and tumor invasiveness in human osteosarcoma cell lines" PROCEEDINGS OF THE AMERICAN ASSOCIATION FOR CANCER RESEARCH ANNUAL MEETING, no. 41, March 2000 (2000-03), page 434, XP008060502 & 91ST ANNUAL MEETING OF THE AMERICAN ASSOCIATION FOR CANCER RESEARCH.; SAN FRANCISCO, CALIFORNIA, USA; APRIL 01-05, 2000 ISSN: 0197-016X
- LEE JAE-YOUNG ET AL: "Galectin-3 expression in the development of gastric carcinomas" PROCEEDINGS OF THE AMERICAN ASSOCIATION FOR CANCER RESEARCH ANNUAL MEETING, no. 41, March 2000 (2000-03), page 518, XP008060442 & 91ST ANNUAL MEETING OF THE AMERICAN ASSOCIATION FOR CANCER RESEARCH.; SAN FRANCISCO, CALIFORNIA, USA; APRIL 01-05, 2000 ISSN: 0197-016X

## Description

### FIELD OF THE INVENTION

The present invention relates to a method for predicting cancer in a simple and easy manner. More particularly, the present invention relates to a method for predicting and/or diagnosing the occurrence of stomach cancer or the risk of contracting stomach cancer by measuring the concentration of galectin-3 in blood, which changes before the occurrence of the cancer in a patient. The present invention is particularly useful for (1) a patient having stomach cancer, but no subjective symptom, (2) a patient having a benign tumor, a chronic inflammation or gastritis, but no subjective symptom, and (3) a normal person. The patients in the state of above (1) and (2) are capable of predicting the occurrence of cancer or the risk of contracting a cancer with the method according to the present invention, and may have a more precise medical examination, which helps diagnosis of the cancer in early stage.

### BACKGROUNDS OF THE INVENTION

The detection of a cancer in early stage is most important in healing the cancer. In most case, the subjective symptom due to a cancer is revealed after the cancer is complicated. The medical check up examination for cancer in hospital is generally not easy for ordinary person, and this makes it difficult to find out the cancer in early stage. There is a continuing need for a method to predict the occurrence of cancer or the risk of contracting a cancer in a simple and easy manner so that ordinary person can self-examine the occurrence of cancer conveniently and in early stage of the cancer development.

To develop simple and easy method to diagnose and remedy cancer, immunologists have tried to find out tumor-specific transplantation antigens (TSTA). However, due to the characteristic fact that the tumor is derived from the normal cell of a human, the TSTA is not identified except for some malignant epithelioma. Meanwhile, it is also tried to use tumor-associated transplantation antigens (TATA) such as an alpha-fetoprotein (AFP) and a carcinoembryogenic antigen (CEA), which exists in normal cell, but increases in tumor cell, for diagnosing the cancer. However, the over-manifestation of TATA occurs only after the tumor is complicated. Therefore, the method for diagnosing tumor with TATA is not much helpful in early diagnosing the cancer.

Galectins is a β-galactoside-binding lectin, and exists in most plant and animal including a poriferan, an eelworm, mammamlia, etc, and has high affinity to a laminin which is an extracellular matrix (ECM) of animal, and known to have close relation with the metastasis of tumor [26, 27]. In addition, it is also known that the galectins have close relation with the growth and transformation of a cell, and metastasis of a tumor. Until now, nine kinds of galectin are identified, and form a galectin family. The galectins have molecular weight of 14-36kDa, and exists in monomer or dimer forms. The galectin-1, galectin-3, and galectin-8 exist in various internal organs, and galectin-2, galectin-4, galectin-5 and galectin-7 exist in specific internal organs or cells [2, 10, 15, 17, 25].
Galectin-3 is also called as CBP-35, Mac-2, ε BP, RL-29, L-34, L-31 etc, and discovered by Ho and Springer [12] in the macrophage activated by thioglycollate, and is a protein having molecular weight of about 26-32 kDa [27]. The galectin-3 relates with the growth, differentiation, malignant degeneration, and embryo formation of a cell, and also relates with the hypersensitivity reaction of a cell mediated with IgE, and plays important role in the binding between cells, and between cells and matrix. The galectin-3 accelerates the uptake of the calcium ion in human Jurkat T-cell [8], and has relation with the apoptosis of a T-cell [29]. Galectin-3 can exist in the form of ribonucleoprotein (RNP)/galectin-3 complex, and effects the pre-mRNA substrate and the RNA splicing process [7].

It is also known that the galectin-3 can be released from a cell without signal peptide [31]. As the biological materials having the same releasing property, interleukin-1 (IL-1) and fibroblast growth factor (FGF) are known, but the precise releasing mechanism is not identified yet. This releasing property is quietly different form that of general soluble protein in eukaryotic cell.

The galectin-3 protein is known to have close relation with malignant tumor, and occurs in colon cancer, skin cancer, thyroid gland cancer, breast carcinoma etc [5, 6, 9, 14, 18, 19, 20, 23, 24, 28]. There are much less research in the field of stomach cancer than colon cancer, lung cancer, breast carcinoma and malignant melanoma. The galectin-3 does not manifest in the normal liver cell, but manifests in liver cancer and cardiac cirrhosis [13]. In case of the thyroid gland cancer, it is known that the manifestation of the galectin-3 can be used as a marker before cancer operation [20]. In case of stomach cancer, it is reported that the galectin-3 does not manifest in normal stomach tissue, but manifests in the stomach cancer [30]. In summary, the heretofore researches indicate that the manifestation of galectin-3 has close relation with the malignant tumor.

It has also been reported [14a] that the serum concentration of galectin-3 is significantly elevated in patients with breast, gastrointestinal, lung, or ovarian cancer, melanoma, and non-Hodgkin's lymphoma.

### SUMMARY OF THE INVENTION

In view of the foregoing, I identified the relation of manifestation of galetin-3 and development of tumor, and discovered the over-manifestation of galetin-3 in the stage of a benign tumor or a chronic inflammation and gastritis, which is a former or an initial stage of tumor development. Thus, the present invention is directed to the simple and easy method of using the over-manifestation of galetin-3 in the former and initial stage of tumor development in stomach cancer.

Therefore, it is an object of the present invention to provide a method for diagnosing and/or predicting the occurrence of stomach cancer or the risk of contracting stomach cancer in the former and initial stage of tumor development by measuring the concentration of galectin-3 in blood, which is over-manifested in the stage of a benign tumor (adenoma) or a chronic inflammation which is the former or initial stage of the malignant tumor (carcinoma). The user recognizing the occurrence of stomach cancer or the risk of contracting stomach cancer with the method according to the present invention may have a more precise medical examination in hospitals, which results in the early and exact diagnosis of stomach cancer.

In other words, the method according to the present invention may help the user to self-examine his or her risk of contracting stomach cancer in the initial stage of tumor development, and contributes to the early finding of stomach cancer, in the early stage of tumor development. Therefore, it is other object of the present invention to provide a method for diagnosing and/or predicting the occurrence of stomach cancer of user having no subjective symptom.

To accomplish these and other advantages, the present invention provides a method of diagnosing and/or predicting the occurrence of stomach cancer or the risk of contracting stomach cancer as dutlined in the appended claims.

### BRIEF DESCRIPTION OF THE DRAWINGS & PHOTOGRAPHS

A more complete appreciation of the invention, and many of the attendant advantages thereof, will be readily apparent as the same becomes better understood by reference to the following detailed description when considered in conjunction with the accompanying drawings and photographs, wherein:
Fig. 1A is a microscope photograph showing the manifestation of galectins-3 at Auerbach plexus surrounded by normal cell (magnifying ratio: 200);
Fig. 1B is a microscope photograph showing the manifestation of galectins-3 at semi-differentiated signet ring cell (magnifying ratio: 200);
Fig. 2A is a microscope photograph showing the manifestation of galectins-3 at intestinal metaplasia (magnifying ratio: 100);
Fig. 2B is a microscope photograph showing the manifestation of galectins-3 at tubular carcinoma (magnifying ratio: 200);
Fig. 3 is a microscope photograph showing the manifestation of galectins-3 at well-differentiated tumor tissue and semi-differentiated signet ring cell (magnifying ratio: 200);
Fig. 4 is a graph showing the ratio of 5 years survival according to the degree of the manifestation of galectins-3; and
Fig. 5 is a graph showing the concentration of galectins-3 in blood of normal human, patient having stomach cancer, and patient having chronic gastritis. The concentrations of galectins-3 in blood sample, which is diluted successively, were represented by absorbance measured by the ELISA method. In Fig.5, x-axis represents dilution ratio of the blood sample, and y-axis represents absorbance at 490nm. Each mark in Fig.5 is depicted to represent mean ± standard deviation.
Fig.6 is a plan view of an assay strip forming the kit according to an embodiment of the present invention.

### DETAILED DESCRIPTION OF THE INVENTION

For a better understanding of the present invention, reference will now be made in detail to the following disclosures and appended claims.

The present invention provides a method for diagnosing and/or predicting the occurrence of stomach cancer or the risk of contracting stomach cancer. Heretofore, the cancer is diagnosed by detecting the concentration of the tumor-associated transplantation antigens (TATA), which exists in normal cell, but increases in tumor cell. However, the over-manifestation of TATA occurs when the tumor is complicated, i.e. after the initial stage of the tumor development. Therefore, the method for diagnosing tumor with TATA is not much helpful in early diagnosing the cancer.

In contrast, the present invention is directed to a new concept for early diagnosing the tumor development. The method according to the present invention is to diagnose and/or predict the risk of contracting a cancer with an antigen which is over-manifested in blood before the development of the malignant tumor(carcinoma), rather than using an antigen which is over-manifested after the development of the carcinoma. The antigen, which is used in the present invention, is gacectin-3.

According to the present invention, the suitable cancer screening antigen is the galectin-3. The galectin-3 is not manifested in normal tissue such as normal stomach tissue, liver tissue and thyroid gland, but over-manifested in the stage of a benign tumor (adenoma) or a chronic inflammation which is the former stage of the malignant tumor(carcinoma). Thus, the galectin-3 can be used for early diagnosing the stomach cancer.

The method of using the galectin-3 in predicting the occurrence of stomach cancer includes the steps of determining the concentration of the galectin-3 in the blood sample of a user via antigen-antibody reaction in a method as outlined in the appended claims. The galectin-3 is not manifestes in normal blood, but over-manifested in the stage of the adenoma or the chronic inflammation, which is the former stage of the carcinoma, and the over-manifestation is maintained in the stage of the carcinoma. Thus, if the concentration of the galectin-3 in the blood sample of a user is greater than that of the normal human, the user might be in the initial or former stage of the disease, and there is high probability of contracting stomach cancer.

Because the method according to the present invention diagnoses cancer by determining the concentration of the galectin-3 in blood sample rather than directly examining the tissues of stomach, liver and thyroid gland, the discrimination of the specific type of cancer, such as stomach cancer cannot be complete. However, if the test result is positive, the user may have a more precise medical examination, which results in the early and exact diagnosis of stomach cancer.

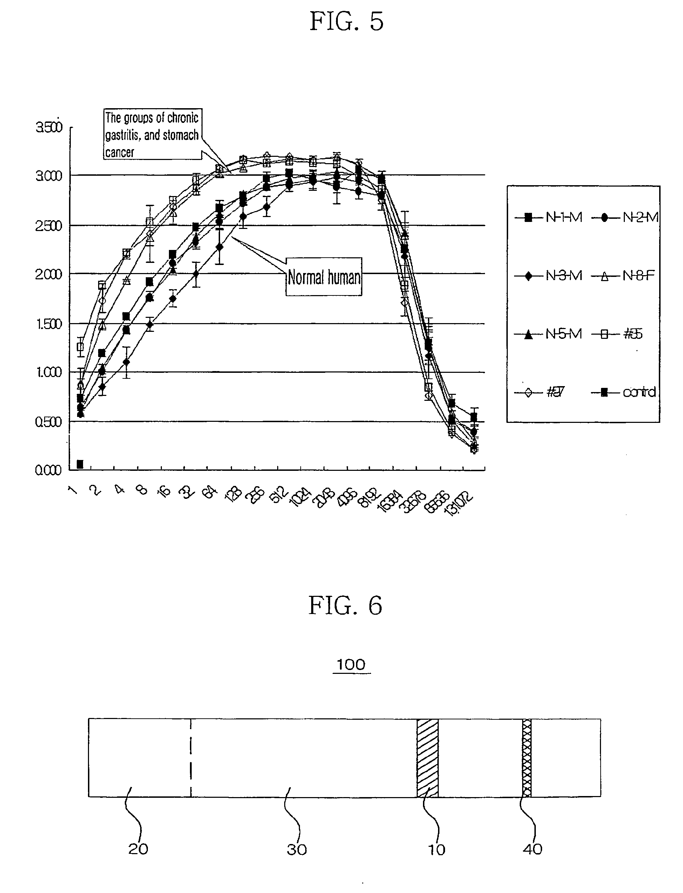

The method of the present invention is to determine galectin-3 in the blood sample by using an assay strip as outlined in the appended claims. As shown in Fig. 6, the simplest configuration of the assay strip 100 includes a reaction part 10, a sample injection part 20, and a membrane 30 for providing passage from the sample injection part 20 to the reaction part 10. The assay strip 100 can be protected by a plastic case having at least one window for observing the reaction part 10 and for injecting the sample.

In one embodiment, a capture antibody of the galectin-3 is immobilized to the reaction part 10, and a blood sample including the antigen (galectin-3) and gold-conjugated tracer antibody of the galectin-3 are moved trough the membrane 30 from the sample injection part 20 to the reaction part 10. In this embodiment if the galectin-3 is over-manifested in the blood sample, the more galectin-3 reacts with the gold-conjugated tracer antibody, and the greater amount of the antibody-antigen complex is captured on the reaction part 10, which results in the color change of the reaction part 10. On the contrary, if the galectin-3 is not over-manifested in the blood sample, the gold-conjugated tracer antibody cannot react with the antigen, thus cannot be captured on the reaction part 10. In this case, the color of the reaction part 10 cannot be changed. Therefore, a user can determine the concentration of the galectin-3 according to the color change of the reaction part 10.

The gold-conjugated tracer antibody can be directly mixed with the blood sample and injected onto the sample injection part 20. Alternatively, the gold-conjugated tracer antibody can be positioned between the sample injection part 20 and the reaction part 10 in a dried state. In latter case, the dried gold-conjugated tracer antibody melts when the blood sample moves from the sample injection part 20 to the reaction part 10, and reacts with the galectin-3 in the blood sample.

In other embodiment, galectin-3 is immobilized to the reaction part 10, and a blood sample including galectin-3 and gold-conjugated tracer antibody of the galectin-3 are moved trough the membrane 30 from the sample injection part 20 to the reaction part 10. When the blood sample including the galectin-3 and gold-conjugated tracer antibody reaches the reaction part 10, the galectin-3 immobilized onto the reaction part 10 and the galectin-3 in the blood sample competitively react with the gold-conjugated tracer antibody. Therefore, if the galectin-3 is over-manifested in the blood sample, the galectin-3 immobilized onto the reaction part 10 cannot react with the gold-conjugated tracer antibody. Thus, the color of the reaction part 10 cannot be changed. On the contrary, if the galectin-3 is not over-manifested in the blood sample, the galectin-3 immobilized onto the reaction part 10 captures large amount of the gold-conjugated tracer antibody, which results in the color change of the reaction part 10.

In summary, the reaction part 10 includes the capture antibody of galectin-3 or galectin-3 immobilized thereon so that the color of the reaction part 10 is determined according to the concentration of galectin-3 in the blood sample when the blood sample including galectin-3 and the gold-conjugated tracer antibody of the galectin-3 reaches to the reaction part 10 trough the membrane 30.

To clearly detect the color change of the reaction part 10, the blood sample can be decolorized according to the conventional method, such as mixing the blood sample with an oxidant. Alternatively, a filter for filtering the coloring matter in the blood sample can be formed between the sample injection part 20 and the reaction part 10. Preferably, a conventional control line 40 can be formed on the membrane 30 at the opposite end of the sample injection part 20, and the control line 40 is designed to change its color when the blood sample arrives to the control line 40, which makes the user to confirm the test is completed.

Generally, even the normal tissue includes small amount of galectin-3. Therefore, the color change of the assay strip should be controlled so that the color change should occur when the concentration of the galectin-3 in the blood sample is greater than that in the normal blood. In addition, the color change due to the concentration of the galectin-3 depends on the dilution ratio of the sample (See following Example). Therefore, the sample dilution ratio should be controlled so that the color of the reaction part cannot be changed with the normal blood, and can be changed with the abnormal blood. The dilution ratio can be properly controlled according to the configuration of the assay strip.

In order to more fully illustrate the preferred embodiments of the present invention, the following detailed examples are given.

### Example

### 1. Material and method.

### (1). Preparation of stomach cancer tissue and blood

100 stomach cancer tissue slides were obtained from the Aju University Hospital in Republic of Korea. The tissue slides were collected during the gastrectomy operations of the 100 patients who were diagnosed to have stomach cancer by pathological diagnosis during 1994-1996. The tissue slides consisted of tissues under advanced carcinomas, early gastric carcinomas (EGC), precancerous lesions, and normal gastric mucosal tissues. The normal peripheral blood were obtained from normal person who are selected in random, and the peripheral blood of patients having stomach cancer or gastritis were obtained from the Aju University Hospital and Catholic University Vincent Hospital in Republic of Korea.

### (2). Immunohistochemical staining of the galectin-3 protein in stomach cancer tissue

In order to remove the paraffin in the prepared slides and to hydrate the slides, the tissue slides were dipped into xylene solution for 5 minutes, and the dipping process was repeated 3 times. Then the slides were successively dipped into 100% ethanol solution, other 100% ethanol solution, 90% ethanol solution, 80% ethanol solution, and 70% ethanol solution each for 5 minutes. Then the slides were dipped into distilled water for 10 minutes, and 3% hydrogen peroxide solution for 5 minutes, and washed with PBS containing 1% Triton X-100, 3 times each for 5minutes. The washed slides were moved into a humidified chamber in order to prevent non-specific binding reactions, and treated with 10% normal goat serum, and reacted for 30minutes at room temperature.

After the reaction was complete, the culture supernatant of cell strain producing M3/38 monoclonal antibody (rat IgG, 1 st antibody) was added to the humidified chamber, and maintained at 4°C for over-night, and washed with PBS containing 1% Triton X-100 3 times each for 5minutes. As the 2nd antibody, a horse radish peroxidase (HRP)-conjugated goat anti-rat IgG (Pierce co.) was diluted with PBS containing 3% BSA with the dilution ratio of 1: 800, and was added to the humidified chamber, and reacted for 1 hour at room temperature, and washed with PBS containing 1% Triton X-100 3 times each for 5minutes. Then the tissue is reacted with 3,3'-diaminobenzidine (DAB), and washed, and stained with Harris' hematoxylin as control, and dehydrated.

### (3). Measurement of concentration of the galectin-3 in peripheral blood

The concentration of the galectin-3 in peripheral blood was measured as follows with ELISA (Enzyme-linked Immunosorbent Assay).

100µl of buffer solution including same amount of 0.5M carbonate and 0.5M bicarbonate (pH = 9.6) was coated on two 96 well plates, respectively. Then, 50µl of the buffer solution and 50µl of the blood sample including antigen (galectin-3) was coated onto one of the 96 well plates. The mixture was mixed well, and 100µl of the mixture was transferred to the 2nd 96-well plate to dilute the sample 2 times. By repeating the dilution process, the mixture was diluted to 2048 times, and in some experiment, diluted to 131072 times.

The diluted mixtures were maintained at 4°C for over-night to coat the antigen(galectin-3) onto the 96-well plate. Then the coated antigen was washed with washing solution (PBS+ 0.05% Tween-20) 3 times, and blocked with PSB containing 3% BSA, and cultured for 1 hour at 37°C. Then the microplate was washed.

Then, the 1st antigen was added to the coated antigen. In detail, the prepared monoclonal antibody of the galectin-3 was diluted with EIA buffer solution having the following composition with the dilution ratio of 1:1, and inoculated to the well(1 00 µl/well), and incubated for 2 hours at 37°C, and washed.

**< Composition of EIA buffer solution (100ml) >**

| | |
|---|---|
| 0.01 M tris-HCl buffer solution | 75ml |
| Tween-20 | 0.1ml |
| Fetal bovine serum | 25ml |
| EDTA | 200mg |
| Thimerosal | 5mg |

Then the 2nd antibody was reacted. In detail, goat anti rat lg G-HRP was diluted with the dilution ratio of 1:1,500, and inoculated to the well (100 µl/well), and incubated for 2 hours at 37°C, and washed. The next step was to inoculate chromogen solution to the well (100 µl/well). At this time, a stock solution was prepared so that the final concentration of OPD was 0.5 mg/mℓ, and the buffer solution of the OPD substrate consisted of 0.05M sodium citrate and 0.05M Na₂HPO₄ (pH 5.0). The HRP substrate was prepared by mixing 1 mℓ of OPD stock (ortho-phenylenediamine stock solution), 9 mℓ of OPD buffer solution, and 3.3 µℓ of 30% H₂O₂, and the mixture was incubated for 1 hour at room temperature. Then the reaction was stopped by inoculation of 2.5M H₂SO₄ (50 µℓ /well), and the light absorbance of the plate at 490 nm was measured.

### (4). Pathological interpretation

The manifestation of galectin-3 at Auerbach plexus disposed in stomach was regarded as control. The pathological diagnosis was interpreted according to the following criteria.
(-): galectin-3 is not manifested
(1+): galectin-3 is manifested in the tumor cell of less than 5%
(2+): galectin-3 is manifested in the tumor cell of 5-50%
(3+): galectin-3 is manifested in the tumor cell of more than 50%

### (5). Grouping and statistical analysis

The four criteria are divided into 2 groups as follows for comparing the pathological parameters according to the manifestation of the galectin-3.
A group: 3+
B group: -, 1+, 2+

Pathological parameters, such as a depth of invasion, number of lymph node metastasis, and TNM stage according to the UICC classification method between the two groups were analyzed with the Chi-square test method, and the ratios of survival of the two groups according to the manifestation of the galectin-3 was determined with Kaplan-Meier method. When P value was less than 0.05, the data is regarded as statistically useful.

### 2. Result

### (1). Immunohistochemical staining

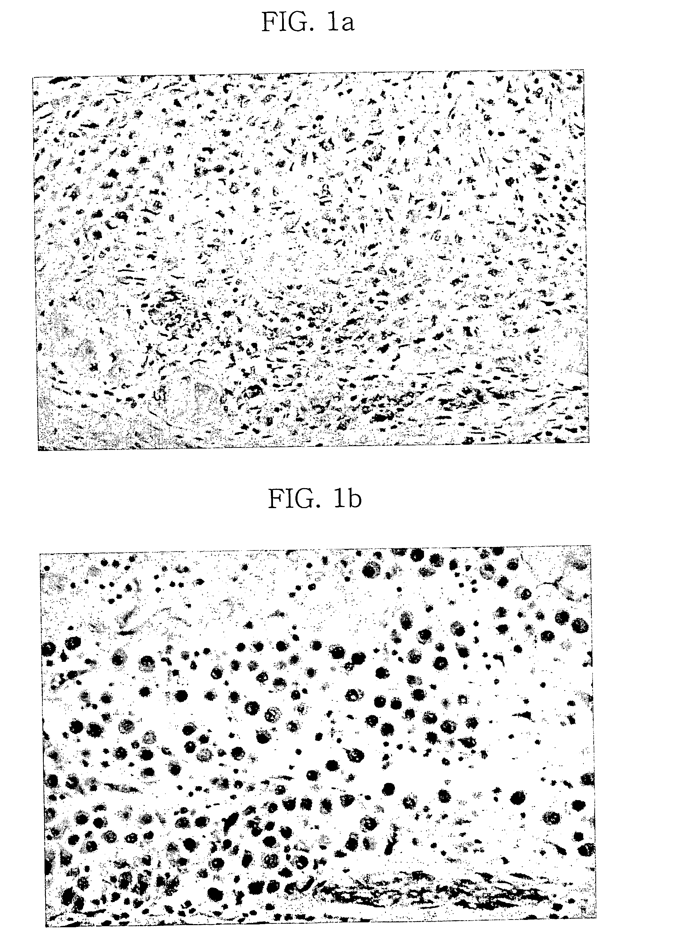

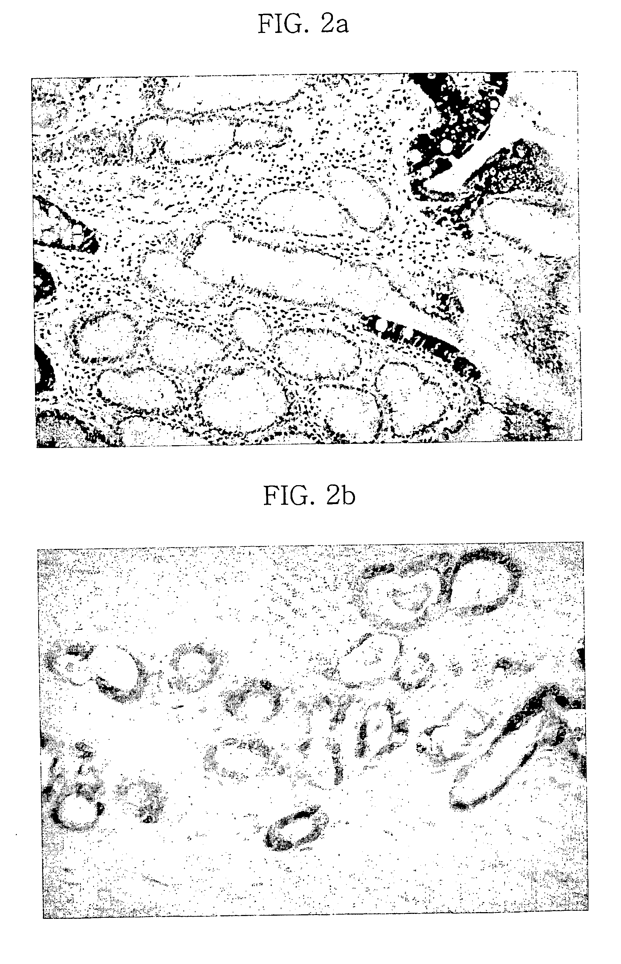

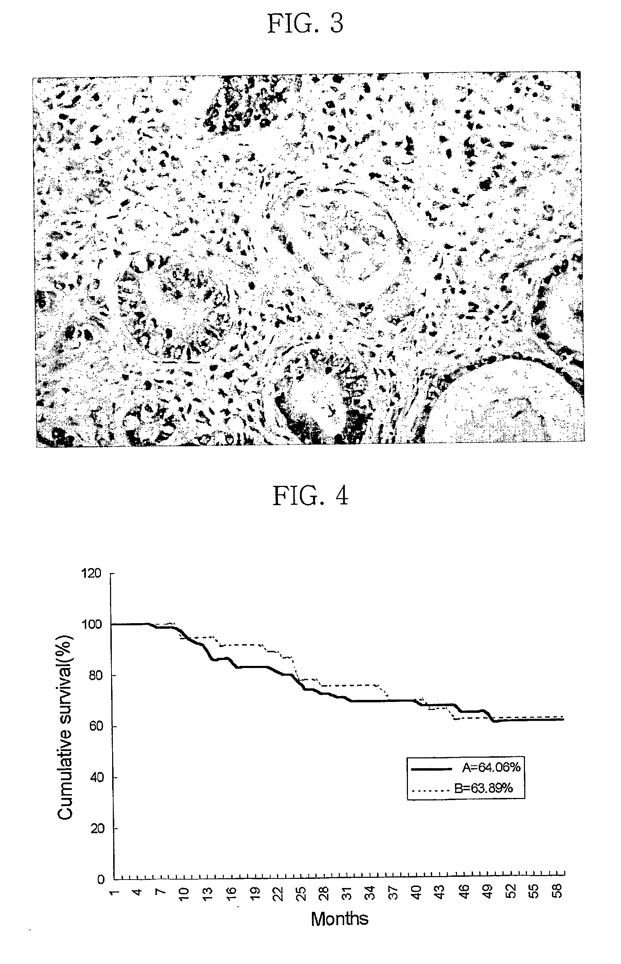

From the manifestation of the galectin-3 at Auerbach plexus (See Figs. 1A and 1B), it is clear that galectin-3 was seldom manifested in normal cell, and only small amount of the galectin-3 was manifested at the mucosal neck portion. As shown in Figs. 2A and 2B, the galectin-3 is more strongly manifested in the stage of intestinal metaplasia and adenoma which are former stages of the tumor. As shown in Fig. 3, the galectin-3 is widely manifested in the tumor tissue, but the degree of the manifestation increases with the degree of differentiation of a cell. Thus the degree of the manifestation at the stage of the differentiation of a cell is greater than that of the stage of the malignant tumor.

### (2). Comparison of the two groups according to the degree of manifestation of the galectin-3

100 patients having stomach cancer included 72 men and 28 women, and their average age was 53.7 (age range: 27-78). Among them, 64 people were classified into the group A, and 36 people were classified into group B. The pathological parameters of the two groups were compared, and the results are shown in Table 1. In case of group B, the numbers of intestinal type and diffuse type are almost same, but in case of group A, the number of intestinal type was greater than that of the diffuse type (p<0.05). In case of the depth of invasion, the ratio of T3 was the greatest in both of the two groups (p<0.05), and the number of lymph node metastasis was in the range of 1-6(p<0.05).

The ratios of stage III among stage I-IV were greatest, but the p-value was greater than 0.05, and the test results were regarded as not useful statistically.

**Table 1: Pathological analysis of the patient groups according to the manifestation of the galactin-3.**

| Pathological parameter | Group A Number(%) | Group B Number(%) | P-value |
|---|---|---|---|
| Lauren | | | |
| Intestinal Type | 52(81.3) | 17(47.2) | < 0.05 |
| Diffuse Type | 12(18.8) | 19(52.8) | |
| Depth of Invasion | | | |
| T1 | 8(12.5) | 3(8.3) | < 0.05 |
| T2 | 6(9.4) | 7(19.4) | |
| T3 | 50(78.1) | 23(63.9) | |
| T4 | 0(0) | 3(8.3) | |
| No. of Positive LNs | | | |
| 0 | 17(26.6) | 3(8.3) | < 0.05 |
| 1-6 | 22(34.4) | 20(55.6) | |
| 7-15 | 14(21.9) | 10(27.8) | |
| more than 16 | 11 (17.2) | 3(8.3) | |
| Stage | | | |
| I | 8(12.5) | 3(8.3) | 0.648 |
| II | 16(25.0) | 6(16.7) | |
| III | 29(45.3) | 20(55.6) | |
| IV | 11(17.2) | 7(19.4) | |

| | | | |
|---|---|---|---|
| The degree of manifestation of galectin-3 of Group A(n=64): 3+ The degree of manifestation of galectin-3 in Group B(n=36): -,1 +, 2+ | | | |

As shown in Fig.4 showing the ratio of 5 years survival according to the manifestation of the galectin-3, the ratio of 5 years survival of the Group A was 64.06%, and the ratio of 5 years survival of the Group B was 63.89% (p value = 0.9153). Thus, it is found that the manifestation of the galectin-3 has no relation with the expected life time of the patients.

### (3). Detection of the galectin-3 at peripheral blood.

The peripheral blood was obtained from the groups of normal human, patient having chronic gastritis, patient having benign tumor, and patient having stomach cancer, respectively. The concentrations of galectin-3 were measured, and the results are depicted in Fig. 5. As shown in Fig.5, the manifestations of the galectin-3 of the groups of chronic gastritis, benign tumor, and stomach cancer were greater than that of normal human. Thus, the galectin-3 can be used more effectively as a cancer anticipating or predicting antigen rather than cancer diagnosing agent. The discriminating power of the manifestations was maximized with 100 times dilution of the blood sample.

### 3. Discussion

From the above-described experiment, the degree of manifestation of the galectin-3 at each stage of the tumor development was determined, and its relation with the tumor developments was discovered. In sum, the galectin-3 seldom manifests in normal cell, but strongly manifests in the stage of intestinal metaplasia and adenoma which are the former stages of the tumor. The most unexpected result obtained from the experiment is that the galectin-3 more strongly manifests in the stage of intestinal metaplasia and adenoma than in the stage of the tumor. The manifestation of the galectin-3 is more stronger in the well differentiated tumor cell than in undifferentiated tumor cell. This means that the manifestation of the galectin-3 increases with the initiation of intestinal metaplasia and adenoma, and is maximized during the stages of intestinal metaplasia and adenoma, and maintained when the intestinal metaplasia and adenoma are developed to tumor.

From the pathological parameter analysis, the manifestation of the galectin-3 had little relation with the depth of invasion and the number of lymph node metastasis. This result is similar to the result of research of breast carcinoma, and different from the result of research of colon cancer, lung cancer, melanoma etc, in which the manifestation of the galectin-3 has close relation with development of malignant tumor. From the experiment to the stomach cancer, it is found that the manifestation of the galectin-3 has little relation with the ratio of survival of patients (p=0.9153), which means that the galectin-3 is not so effective as a diagnostic marker specific to the malignant tumor.

However, in the present invention, the galectin-3 is mainly used as a diagnosing agent of the intestinal metaplasia and adenoma rather than the malignant tumor. The galectin-3 is expected to have a role in the initial or middle stages of the development of carcinogenesis since the galectin-3 has mainly manifested at the former or initial stage of the development of the stomach cancer. Therefore, the manifestation of the galectin-3 is useful as a screening marker for early predicting of stomach cancer and the probability of contracting stomach cancer.

### 4. Conclusion

It is reported that the manifestation of the galectin-3 increases in the malignant tumor such as colon cancer, thyroid gland cancer, lung cancer and black epithelioma, and decreases in the breast cancer. In case of stomach cancer, it is reported that the galectin-3 seldom manifest in the normal tissue of stomach, but manifest in tissue of the stomach cancer [30]. In the above report, the manifestation of the galectin-3 in normal stomach tissue and stomach cancer was studied, but the manifestation of the galectin-3 during development of cancer was not established, thus the use of galectin-3 as an antigen for early predicting of tumor was not developed in the prior report. In contrast, the manifestations of the galectin-3 in normal tissue and blood, former stage of stomach cancer and initial stage of benign tumor and complicated malignant tumor were studied in the present invention, and it is found that the over-manifestations of the galectin-3 can be used as a signal for initiation of the malignant tumor, namely, a signal for detecting the former and initial stages of the tumor development.

In this disclosure, there is shown and described only the preferred examples of the invention, but, as aforementioned, it is to be understood that the invention is capable of use in various other combinations and environments and is capable of changes or modifications within the scope of the inventive concepts as expressed herein.

### REFERECES

1. Barondes, S.H., Cooper, D.N.W., Gitt, M.A. and Leffler, H. : Galectins : structure and function of a large family of animal lectins, J. Biol. Chem., 269 : 20807-20810, 1994.
2. Barondes, S.H., Castronovo, V., Cooper, D.N.W., Cummings, R.D., Drickamer, K., Feizi, T., Gitt, M.A., Hirabayashi, J., Hughes, C., Kasai, K-I., Leffler, H., Liu, F-T., Lotan, R., Mercurio, A.M., Monsigny, M., Pillai, S., Poirer, F., Raz, A., Rigby, P.W.J., Rini, J.M. and Wang, J.L. : Galectins : a family of animal β-galactoside-binding lectins, Cell, 76 : 597-598, 1994.
3. Blaser, C., Kaufmann, M., Muller, C., Zimmermann, C., Wells, V., Mallucci, L. and Pircher, H. : β-galactoside-binding protein secreted by activated T-cells inhibits antigen-induced proliferation of T-cells, Eur. J. Immunol., 28 : 2311-2319, 1998.
4. Bresalier, R.S., Yan, P-S., Byrd, J.C., Lotan, R. and Raz, A. : Expression of the endogenous galactose-binding protein galectin-3 correlates with the malignant potential of tumors in the central nervous system, Cancer, 80 : 776-787, 1997.
5. Bresalier, R.S., Mazurek, N., Stemberg, L.R., Byrd, J.C., Yunker, C.K., Nangia-Makker, P. and Raz, A. : Metastasis of human colon cancer is altered by modifyng expression of the β-galactoside-binding protein galectin-3, Gastroenterology, 115 : 287-296, 1998.
6. Castronovo, V., Van den Brule, F.A., Jackers, P., Clausse, N., Liu, F-T., Gillet, C. and Sobel, M.E. : Decreased expression of galectin-3 is associated with progression of human breast cancer, J. Pathol., 179 : 43-48, 1996.
7. Dagher, S.F., Wang, J.L and Patterson, R.J. : Identification of galectin-3 as a factor in pre-mRNA splicing, Proc. Natl. Acad., 92 : 1213-1217, 1995.
8. Dong, S and Hughes, C. : Galectin-3 stimulates uptake of extracellular Ca2+ in human Jurkat T-cells, FEBS Letters, 395 : 165-169, 1996.
9. Fernandez, P.L., Merino, M.J., Gomez, M., Campo, E., Medina, T., Castronovo, V., Sanjuan, X., Cardesa, A., Liu, F-T. and Sobel, M.E. : Galectin-3 and laminin expression in neoplastic and non-neoplastic thyroid tissue, J. Pathol., 181 : 80-86, 1997.
10. Gitt, M.A., Wiser, M.F., Leffler, H., Herrmann, J., Xia, Y-R., Massa, S.M., Cooper, D.N.W., Lusis, A.J. and Barondes, S.H. : Sequence and mapping of galectin-5, a β-galactoside-binding lectin, found in rat erythrocytes, J. Biol. Chem., 270 : 5032-5038, 1995.
   11. Hermanek, P and Sobin L.H. : TNM classification of malignant tumors, 4th ed. Geneva : Union Internationale Contrele Cancer (UICC), 1987
12. Ho, M-K. and Springer, T.A. : Mac-2, a novel 32,000 Mr mouse macrophage subpopulation-specific antigen defined by monoclonal antibodies, J. Immunol., 128 : 1221-1228, 1982
13. Hsu, D.K., Dowling, C.A., Jeng, K.C.G., Chen, J-T., Yang, R-Y. and Liu, F-T. : Galectin-3 expression is induced in cirrhotic liver and hepatocellular carcinoma, Int. J. Cancer, 81 : 519-526, 1999
14. Irimura, T., Matsushida, Y., Sutton, R.C., Carralero, D., Ohannesian, D.W., Cleary, K.R., Ota, D.M., Nicolson, G.L. and Lotan, R. : Increased content of an endogenous lactose-binding lectin in human colorectal carcinoma progressed to metastatic stages, Cancer Res., 51 : 387-393,1991
14a. lurisci, I., Tinari, N., Natoli, C., Angelucci, D., Cianchetti, E., lacobelli, S. : Concentrations of galectin-3 in the sera of normal controls and cancer patients, Clin. Cancer Res. 6(4):1389-93, 2000.
15. Leffler, H. and Barondes, S.H. : Specificity of binding of three soluble rat lung lectins to substituted and unsubstituted mammalian β-galactosides, J. Biol. Chem. : 261 : 10119-10126, 1986
16. Lotan, R., Ito, H., Yasui, W., Yokozaki, H., Lotan, D. and Tahara, E. : Expression of a 31kDa lactoside-binding lectin in normal human gastric mucosa and in primary and metastatic gastric carcinomas, Int. J. Cancer, 56 : 474-480, 1994
17. Madsen, P., Rasmussen, H.H., Flint, T., Gromov, P., Kruse, T.A., Honore, B., Vorum, H. and Celis, J.E. : Cloning, expression and chromosome mapping of human galectin-7, J. Biol. Chem., 270 : 5823-5829, 1995
18. Marer, N.L. and Hughes, R.C. : Effects of the carbohydrate-binding protein galectin-3 on the invasiveness of human breast carcinoma cells, J. Cell. Physiol., 168 : 51-58, 1996
19. Ohannesian, D.W., Lotan, D., Thomas, P., Jeesup, J.M., Fukuda, M., Gabius, H-J. and Lotan, R. : Carcinoembryonic antigen and other glycoconjugates act as ligands for galectin-3 in human colon carcinoma cells, Cancer Res., 55 : 2191-2199, 1995
20. Orlandi, F., Saggiorato, E., Pivano, G., Puligheddu, B., Termine, A., Cappia, S., Giuli, P.D. and Angeli, A. : Galectin-3 is a presurgical marker of human thyroid carcinoma, Cancer Res., 58 : 3015-3020, 1998
21. Perrillo, N.L., Marcus, M.E. and Baum, L.G. : Galectins : versatile modulators of cell adhesion cell proliferation and cell death, J. Mol. Med., 76 : 402-412, 1998
22. Raimond, J., Roulex, F., Monsigny, M. and Legrand, A. : The second intron of the human galectin-3 gene has a strong promoter activity down-regulated by p53, FEBS Letters, 363 : 165-169, 1995
23. Sanjuan, X., Fernandez, P.L., Castells, A., Castronovo, V., Van Den Brule, F., Liu, F-T., Cardesa, A. and Campo, E. : Differential expression of galectin-3 and galectin-1 in colorectal cancer progression, Gastroenterology, 113 : 1906-1915, 1997
24. Schoeppner, H.L., Raz, A., Ho, S.B. and Bresalier, R.S. : Expression of an endogenous galactose-binding lectin correlates with neoplastic progression in the colon, Cancer, 75 : 2818-2826, 1995
25. Sparrow, C.P., Leffler, H. and Barondes, S.H. : Multiple soluble β-galactoside-binding lectins from human lung, J. Biol. Chem., 262 : 7383-7390, 1987
26. Woo, H-J., Lotz, M.M., Jung, J.U. and Mercurio, A.M. : Carbohydrate-binding protein 35 (Mac-2), a laminin-binding lectin, forms functional dimers using cysteine 186, J. Biol. Chem., 266 : 18419-18422, 1991
27. Woo, H-J., Shaw, L.M., Messier, J.M. and Mercurio. A.M. : The major non-integrin laminin binding protein of macrophages is identical to carbohydrate binding proein 35 (Mac-2), J. Biol. Chem., 265 : 7097-7099, 1990
28. Xu, X-C., EI-Naggar and Lotan, R. : Differential expression of galectin-1 and galectin-3 in thyroid tumors, Am. J. Pathol., 147 : 815-822, 1995
29. Yang, R-Y., Hsu. D.K. and Liu, F-T. : Expression of galectin-3 modulates T-cell growth and apoptosis, Proc. Natl. Acad. Sci., 93 : 6737-3742, 1996
30. Joo, H-G., and Woo, H-J. : Studies on the expression of galectin-3 in gastric cancer, Kor. J. Immunol., 18 : 583-590, 1996
31. Woo, H-J. : Secretion of macrophage differentiation antigen, Mac-2, Kor. J. Immunol., 15 : 61-68, 1993

## Claims

1. An *in vitro* method of diagnosing or predicting the occurrence of cancer or the risk of contracting a cancer, wherein the cancer is stomach cancer at the former or initial stage of tumor development, comprising the steps of:
(i) providing an assay strip having a reaction part, a sample injection part, a membrane for providing passage from the sample injection part to the reaction part and a control line formed on the membrane at the opposite end of the sample injection part, which is designed to change colons when the blood sample arrives at the control line, wherein either a capture antibody of galectin-3 or galectin-3 is immobilised on the reaction part;
(ii) bringing a blood sample into contact with a gold-conjugated tracer antibody with affinity to galectin-3 either prior to or as the sample moves through the membrane from the sample injection part to the reaction part, wherein the blood sample is diluted with a dilution ratio of 32 to 256; and
(iii) predicting the risk of contracting a cancer according to the colour change of the reaction part.

2. A method according to claim 1, wherein the gold-conjugated tracer antibody is positioned between the sample injection part and the reaction part in a dried state.

## Patentansprüche

1. In vitro-Methode zur Diagnose oder Voraussage des Auftretens von Krebs oder des Risikos sich einen Krebs zuzuziehen, wobei der Krebs Magenkrebs im frühen oder Anfangsstadium der Tumorentwicklung ist, umfassend die folgenden Schritte:
(i) Bereitstellen eines Assaystreifens mit einem Reaktionsteil, einem Probeninjektionsteil, einer Membran, welche die Passage von dem Probeninjektionsteil zu dem Reaktionsteil ermöglicht, und einer Kontrolllinie auf der Membran am gegenüberliegenden Ende des Probeninjektionsteils, welche so entworfen ist, dass sie Kolons ändert, wenn die Blutprobe an der Kontrolllinie ankommt, wobei entweder ein Capture-Antikörper von Galectin-3 oder Galectin-3 auf dem Reaktionsteil immobilisiert sind;
(ii) In-Kontakt-bringen einer Blutprobe mit einem Gold-konjugierten Tracer-Antikörper mit Affinität gegenüber Galectin-3 entweder vor oder während des Durchtritts der Probe durch die Membran von dem Probeninjektionsteil zu dem Reaktionsteil, wobei die Blutprobe in einem Verdünnungsverhältnis von 32 bis 256 verdünnt ist; und
(iii) Voraussage des Risikos sich einen Krebs zuzuziehen anhand der Farbänderung des Reaktionsteils.

2. Methode nach Anspruch 1, worin der Gold-konjugierte Tracer-Antikörper zwischen dem Probeninjektionsteil und dem Reaktionsteil in getrocknetem Zustand positioniert ist.

## Revendications

1. Procédé *in vitro* de diagnostic ou de prédiction de l'apparition d'un cancer ou du risque de contracter un cancer, dans lequel le cancer est le cancer de l'estomac au stade pré-cancéreux, ou au stade initial de développement d'une tumeur, comprenant les étapes consistant à :
(i) prévoir une bande de test présentant une partie réactive, une partie d'injection d'un échantillon, une membrane pour assurer un passage de la partie d'injection d'un échantillon à la partie réactive, et une ligne de contrôle formée sur la membrane à l'extrémité opposée à la partie d'injection d'un échantillon, qui est conçue pour changer de couleur lorsque l'échantillon de sang arrive à la ligne de contrôle, un anticorps de capture de galectine-3 ou de la galectine-3 étant immobilisé sur la partie réactive ;
(ii) amener un échantillon de sang en contact avec un anticorps traceur au conjugué d'or avec une affinité pour la galectine-3 avant que, ou alors que l'échantillon se déplace dans la membrane, de la partie d'injection d'un échantillon à la partie réactive, l'échantillon de sang étant dilué à un taux de dilution de 32 à 256 ; et
(iii) prédire le risque de contracter un cancer en fonction du changement de couleur de la partie réactive.

2. Procédé selon la revendication 1, dans lequel l'anticorps marqueur au conjugué d'or est disposé entre la partie d'injection d'un échantillon et la partie réactive, à l'état sec.
